Europäisches Patentamt

European Patent Office

Office européen des brevets

(19)

(11) Publication number: **0 140 504**

**A1**

(12) **EUROPEAN PATENT APPLICATION**

(21) Application number: **84305535.1**

(22) Date of filing: **14.08.84**

(51) Int. Cl.⁴: **C 12 N 15/00**
**C 07 H 21/04**
**//C12R1/19**

(30) Priority: **15.08.83 US 523502**

(43) Date of publication of application:
**08.05.85 Bulletin 85/19**

(84) Designated Contracting States:
**DE FR GB**

(71) Applicant: **STAUFFER CHEMICAL COMPANY**

**Westport Connecticut 06881(US)**

(72) Inventor: **Nutter, Robert Clair**
**444 30th Street**
**California 94804(US)**

(74) Representative: **Smith, Sydney et al,**
**Elkington and Fife High Holborn House 52/54 High**
**Holborn**
**London WC1V 6SH(GB)**

(54) **Novel plasmid vector.**

(57) Novel plasmid vectors adapted for the transformation of host cells, including a structural gene and control sequences for the transcription and translation thereof are provided. Particular plasmid vectors include control sequences derived from Ti-plasmids of *Agrobacterium tumefaciens*. Plasmids having the control sequence cloned therein are provided. Bacterial strains having therein the plasmids and plasmid vectors are also provided.

EP 0 140 504 A1

# NOVEL PLASMID VECTOR

## Background of the Invention

The transformation of information in the form of naturally occuring or artifically constructed DNA from an originating source into a prokaryotic or eukaryotic cell lies at the heart of genetic engineering. Transformation may be defined as the general process of unidirectional transfer of genetic information in which DNA originating in one cell is taken up and stably maintained by another cell. Such DNA transfers take several forms. Between bacteria, the naturally occurring transfer of information on plasmids is conjugation. Viral DNA may be stably integrated into the bacterial chromosome in lysogenic infection only to be released at a later time in response to a derepressing stimulus. Other naturally occurring forms of transformation include the formation of crown gall tumors in dicotyledonous plants under the influence of tumor-inducing (Ti) plasmids contained in the bacterium Agrobacterium tumefaciens (A. tumefaciens).

The deliberate transformation of prokaryotic cells has quickly become routine. However, the transformation of eukaryotic cells remains a substantial challenge since the genetic fine structure of eukaryotic cells is less well known. It is highly desirable to expand the range of host organisms that may be transformed by placing exogenous DNA into cells of the host. In particular, it is desirable to develop methods and vectors for the transformation of eukaryotic cells including plants and animal cells.

Control of replication, transcription, and translation of exogenous DNA is critical to the expression of characteristics delivered into the host cell in the form of DNA. It is of crucial importance that all these functions be compatible with the enzyme system of the host cell. Thus, the DNA to be employed in transforming the host should include control sequences for the replication of the DNA, control sequences for initiating and terminating the transcription of genes.the expression of which are desired, and control sequences that allow the transcript to be translated and expressed.

A number of reports have described the transformation of plant cells using the bacterium A. tumefaciens carrying plasmids generally known to induce the crown-gall tumor. Such plasmids are generally designated Ti-plasmids. In nature, only dicotyledonous plants may be infected with A. tumefaciens at the site of wounds in the plant's tissue; infection occurs when A. tumefaciens is introduced. In a course of such infections, the plants cells are transformed so that they grow in the absence of certain plant hormones when cultured and may produce opine compounds that are utilized by the infecting A. tumefaciens organism. A gall or plant tumor, composed of these altered cells, forms.

It has been reported that the Ti-plasmid has been isolated and marker DNA sequences such as antibiotic-resistance characteristics have been inserted into the Ti-plasmid. The altered Ti-plasmid is replaced in the A. tumefaciens and plants are infected with the bacteria containing the altered Ti-plasmid. Cells of plants so infected have been found to express the antibiotic-resistance carried by the altered Ti-plasmid.

The transformation of plant cells using A. tumefaciens carrying a modified Ti-plasmid is limited, however, to dicotyledonous plants to which A. tumefaciens is infective. Furthermore, it is necessary to alter the Ti-plasmid so that normal plants can be obtained from the transformed cells.

## Summary of the Invention

The inventor has constructed a novel recombinant plasmid vector that may be used to effect transformation of host cells in addition to those susceptible of A. tumefaciens infections. In general the novel recombinant plasmid vector comprises a plasmid adapted for transformation of the host in that it reproduces in the host cell. Inserted into the plasmid to form the vector is at least one nucleotide sequence coding for a structural gene and control sequences for the transcription and translation of these structural genes. The structural gene may code for any characteristic or product desired to be expressed. Such structural genes may be, for example, without limitation, a complex of genes allowing enhanced amino acid synthesis, a gene or complex of genes conferring resistance to various environmental hazards including toxins, infectious agents, herbicides or antibiotics, a gene or complex of genes coding for a desired product not otherwise produced in the untransformed cell, such as hormones, pharmaceutical precursors, pharmaceuticals, interferon, insecticidal toxins and the like. Structural genes may also include certain characteristics that may be used to identify transformed cells. Such identification characteristics are known within the art as markers and may include specific neculeotide sequences, drug resistances including antibiotic resistance, the ability to synthesis specific growth factors, sensitivity to particular compounds and the like. Positive markers, which are markers that permit the transformed host to survive and grow under conditions that kill untransformed hosts may be used. Such positive markers include antibiotic and drug resistances. Examples of antibiotic resistance includes ampicillin, kanamycin and tetracycline resistance. Drug resistance is further exemplified by the structural genes coding for methotrexate resistant dihydrofoliate reductase which makes the host, particularly eukaryotic hosts, resistant to the metabolic poison methotrexate.

Marker sequences may be used to positively select hosts that have been transformed to have characteristics that are themselves not readily selectable, such as enhanced amino acid synthesis or sensitivity to environmental agents. Thus, methotrexate resistance can be used to screen cells that have been transformed by structural genes inserted into the host along with the structural gene coding for the characteristics of methotrexate resistance.

Control sequences, which are DNA sequences required for the proper transcription and translation of the nucleotide sequence coding for the structural gene are also included in the novel recombinant plasmid. Control sequences include a promoter which includes, as used herein, an RNA polymerase recognition site generally having a nucleotide sequence of thymidine alternating with adenine to give a repeating sequence of TATA-etc., TATAA-etc., TATTTAAA-etc., CATAAA-etc. and a "CAP" site which is believed to provide a site for the binding of RNA polymerase and/or a site for initiation of RNA synthesis. (See DeGreve, H. et al. J. Mol. and App. Gen., (1982) 1:499-511 and Depicker, A. et al., J. Mol. and App. Gen.

(1982) 1:566-573.) The "CAP" site is located in the 3' direction from the TATA etc. sequence and is spaced some 35 nucleotides therefrom.

It is particularly desirable that the promoter function in eukaryotic cells. Thus, the promoter may be derived from a eukaryotic cell or it may be capable of functioning in a eukaryotic cell although it is derived from a prokaryotic source. Alternatively, the promoter may be synthetic. In particular, promoters derived from the Ti-plasmid, which are prokaryotic, are known to function in plant cells. Promoters derived from plasmid pTiA6, pTiAch5 and pTiT37 may be usable as promoters in the recombinant plasmid vector which is described herein. Promoters derived from pTiA6 are particularly preferred.

Other control sequences include a sequence for terminating transcription. In eukaryotic cells these transcription terminating sequences are thought to be 5'-AATAAA-3' or some variation to this sequence. Among the variant sequences that have been described are 5'-AATAAT-3', 5'-AATAAG-3', 5'-GATAA-3', and 5'-ATTAAA-3'. (See DeGreve, H. et al., J. Mol. and App. Gen. (1982) 1:499-511; Depicker, A. et al., J. Mol. and App. Gen. (1982) 1:566-573; Fitzgerald, M., and Shenk, T. Cell (1981) 24:251-260; and Hagenbuchle, O., Cell (1980) 21:179-187.) Transcription terminating sequences are found about 20 nucleotides from the end of all eukaryotic mRNAs. The terminator control sequence whether it is derived from a eukaryotic or non-eukaryotic source should also function in the host cell and in particular, it must function in a eukaryotic cell if the host is eukaryotic. In particular, it is desirable that the terminator function in a plant cell. A terminator that may be used in the recombinant plasmid described herein may also be derived from the plasmid pTiA6.

In general, the structural gene and control sequences will be inserted into a plasmid vector adapted for the transformation of the particular host to be used. Plasmid vectors are plasmids into which an exogenous DNA sequence has been inserted. Plasmids are covalently closed circular pieces of DNA. The plasmid vector is introduced into the host cell and thus is a vehicle for carrying the exogenous DNA into the host. In the present invention, the host to be used is a eukaryotic cell, in particular a plant cell, and more particularly a corn plant or tobacco

5                                                                                    0140504

plant cell.  It has been discovered that pBR327, a plasmid well known as a
vector for transformation of prokaryotic hosts, surprisingly is capable of
replication in plant cells.  Thus, the plasmid vector pBR327 is utilized
as the novel vector described herein.  However, it should be emphasized
that any plasmid vector that is capable of replication in the particular
host to be used is considered to be a suitable vector.

It is an object of the invention to provide a novel recombinant
plasmid comprising a plasmid vector adapted for transformation of a host
into which a DNA sequence comprising a structural gene and control sequences for transcription and translation thereof is inserted.  It is a
further object of the invention to provide a plasmid vector adapted for
the transformation of eukaryotic hosts into which a DNA sequence comprising a structural gene and control sequences for the transcription and
translation thereof has been inserted.  It is yet another object of the
invention to provide a novel plasmid adapted for the transformation of
plant cells, particularly corn plant or tobacco plant cells, into which a
DNA sequence comprising a nucleotide sequence coding for a structural gene
and control sequences for the transcriptions and translation thereof have
been inserted.  It is a further object of the invention to provide a novel
plasmid vector adapted for the transformation of plant cells, particularly
corn or tobacco cells, into which a DNA sequence comprising a nucleotide
sequence coding for a positive marker, particularly methotrexate resistant
dihydrofolate reductase and control sequences for transcription and translation thereof is then inserted.  It is yet a further object of the invention to provide a novel plasmid vector adapated for the transformation of
plant cells, particularly corn plant or tobacco plant cells into which a
positive marker, particularly methotrexate resistant dihydrofoliate reductase and a promoter and transcript terminator sequence for the transcription and translation of said positive marker have been inserted.

## Description of the Drawings

These and other objects of the invention will be better understood with reference to the following figures in which:

Figure 1 is a schematic representation of the steps leading to
the construction of pR11021 (ATCC 39398);

6

Figures 2, 2a and 2B are schematic representations of the steps leading to the construction of pR112048 (ATCC 39399) and

Figure 3 is a schematic representation of the steps leading to the construction of pI11129 (ATCC 39396).

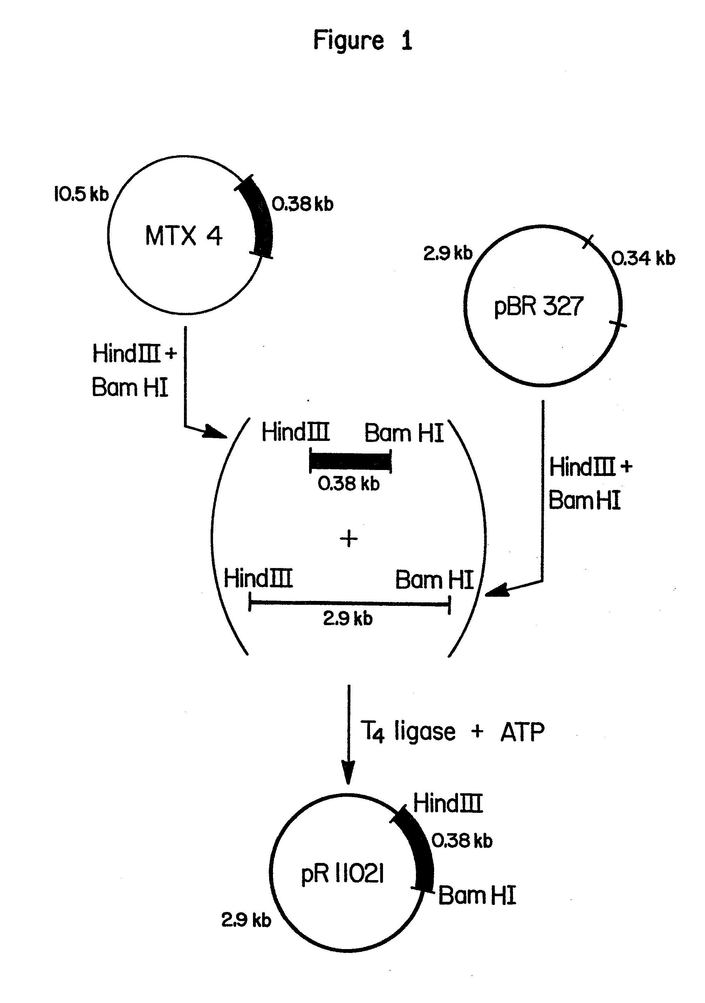

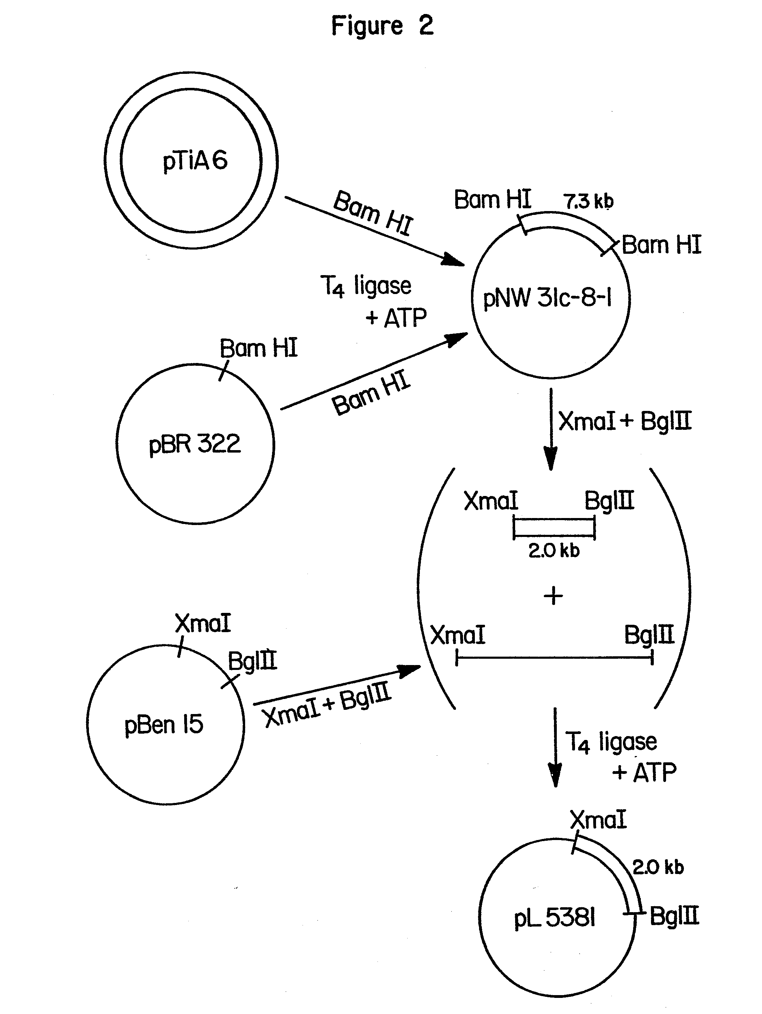

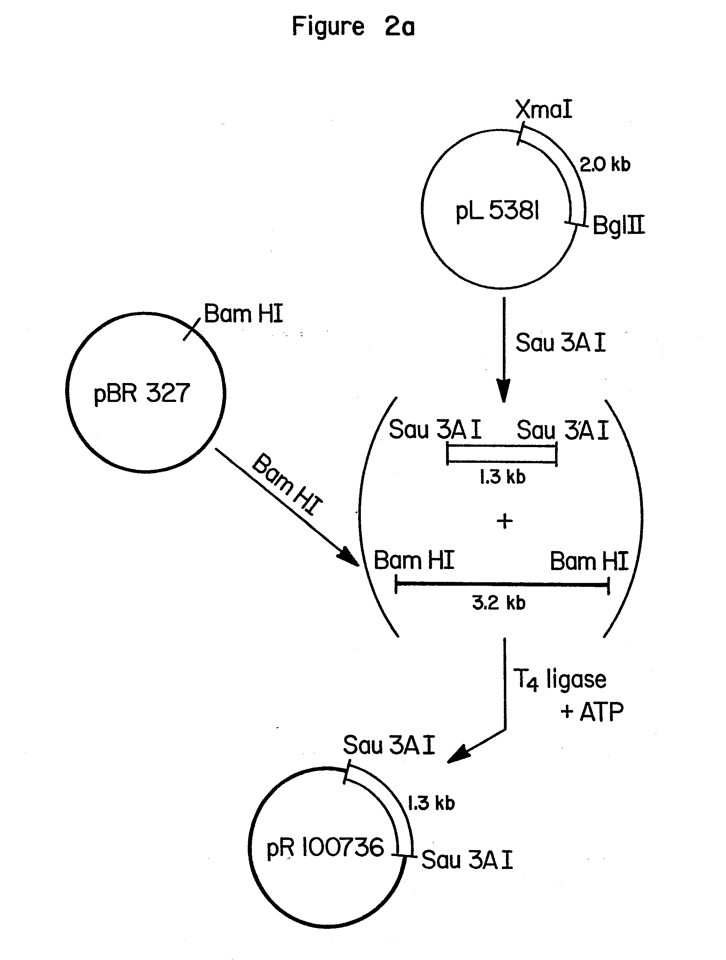

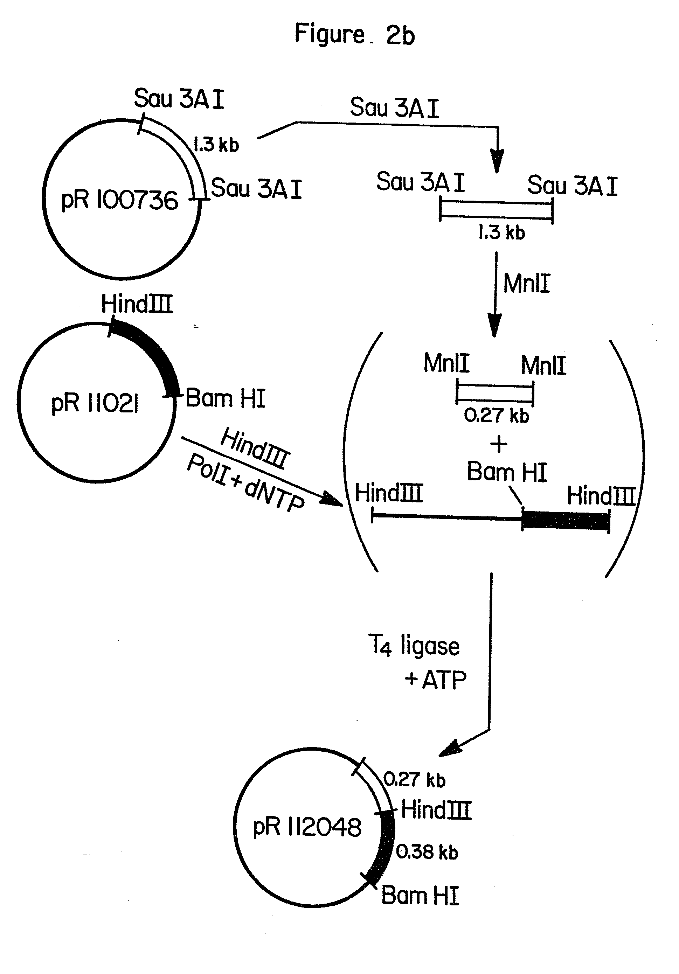

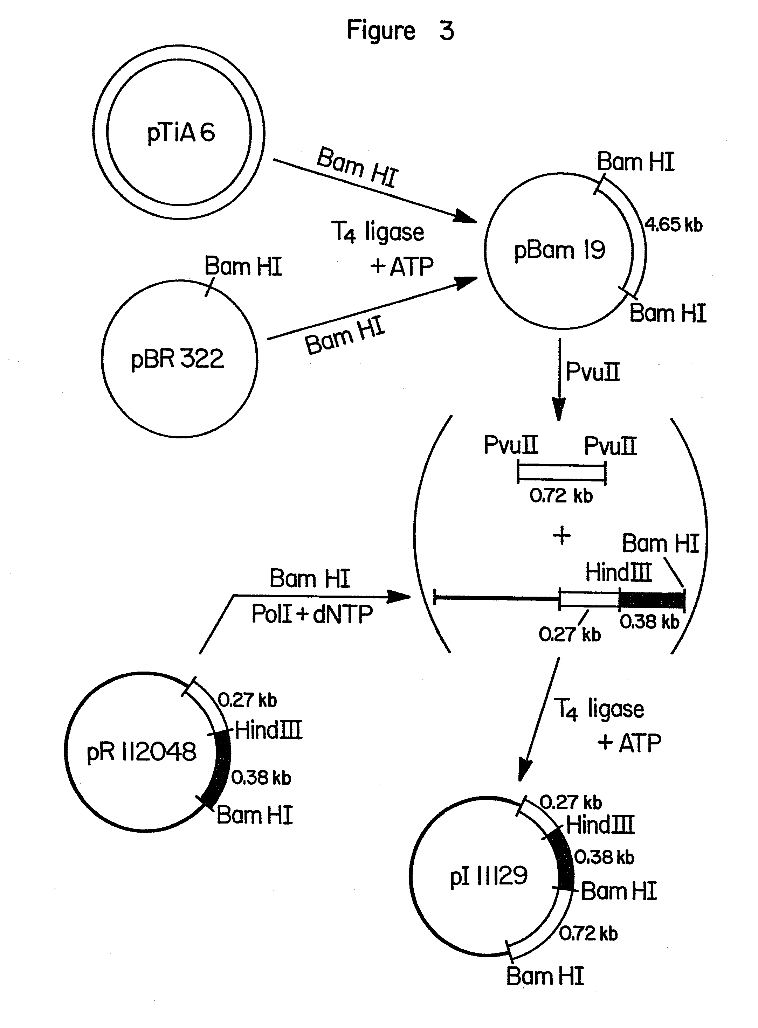

## EXAMPLES

Plasmid pBR327 is known to be stably maintained in corn plant cells in culture. Plasmid pBR327 is isolated from Escherchia coli strain LE392 (hereinafter referred to as LE392) into which it has been previously transformed. pBR327 was isolated from LE392 by the Birnboim-cesium chloride method. Birnboim, H.C., Doly, J. (1979) Nucleic Acid Research, Vol. 7, 1513-1523. The isolated pBR327 is suspended in 10 millimolar (mM) hydroxymethylaminomethane (Tris) and 0.1 mM ethylenediaminotetraacetic acid (EDTA) at pH 8.0 at a stock solution concentration of between 500 micrograms (ug) and 1000 ug of plasmid DNA per milliliter (ml). Working solution concentrations of the plasmid pBR327 DNA are diluted to about 250 ug of plasmid DNA per ml of solution. About 50 ug of pBR327 was digested with Hind III restriction endonuclease and Bam HI restriction endonuclease in the buffer salt solution suggested by the manufacturer (New England Biolabs, Beverly, Massachusetts, hereinafter referred to as NEB). Although the double digest is carried out simultaneously in this example, it can easily be accomplished by sequential digest using Hind III and Bam HI. The restriction endonuclease digest is incubated at 37°C until all DNA appears to be hydrolyzed. The incubation is terminated by heat inactivation. The digested plasmid DNA is first concentrated and then the digested DNA fragments are separated on a 1.5% low melting temperature agarose gel using a buffer of 40 mM Tris, 1 mM EDTA adjusted to pH 8 using glacial acetic acid. The fragments are resolved on the gel after running for 45 minutes at a voltage of between 75 and 100 volts. The Bam HI/Hind III digested pBR327 yields a 2.9 kilobase (kb) fragment and a 0.346 kb fragment. The linear 2.9 kb fragment having one "sticky" Bam HI and one "sticky" Hind III end thus obtained is of suitable purity for use as a vector for cloning of a structural gene.

The structural gene is a DNA sequence coding for methotrexate resistant dihydrofolate reductase ($Mtx^r$DHFR) previously cloned into plasmid MTX4 ATCC 39276 on deposit with the American Type Culture Collection, Rockville, Maryland. Approximately 1.0 ug of this plasmid is digested with Bam HI and Hind III using the same conditions described for the digestion of pBR327. Following concentration, the digested MTX4 DNA is resolved by electrophoresis under the same conditions as described above for pBR327. The Bam HI/Hind III digested MTX4 yields two linear DNA fragments, one 0.37 to 0.38 kb and the other 10.5 to 10.7 kg, each having one Bam HI sticky end and one Hind III "sticky" end, the smaller of the two DNA fragments being the one having the Mtx$^r$DHFR nucleotide sequence.

The agarose gel band contianing the linear 2.9 kb pBR327 vector having Bam HI and Hind III sticky ends and the agarose band containing the Mtx$^r$DHFR nucleotide sequence having Bam HI and Hind III sticky ends are cut out of the gel, placed in a test tube and melted at 65°C at which temperature the agarose liquifies without harming the DNA. The temperature is reduced to 37°C and the solution is diluted with distilled water. Ligase buffer (NEB) adenosine triphosphate (ATP) 0.5 mM and T4 ligase (NEB) are added to the DNA mixture to form a ligation mixture. The final ligation mixture is in a volume of about 100 ul, a volume in which the agar concentration is less than 0.1%. Ligation is carried out for about 2 hours at room temperature at approximately 20-22°C.

Transformation of LE392

Approximately 20 ul of the ligation mixture is mixed with 200 ul of E. coli strain LE392 containing approximatley $10^7$ cells which are previously rendered competent by treatment with calcium chloride in a concentration of between 50 to 100 mM using a method decribed in Cohen et al., P.N.A.S. USA, 69 2110 (1972). The cells and the ligation mixture are held at 0°C for approximately 1 hour followed by heat shock at 37°C for 3 minutes. The calcium chloride concentration is reduced to below 20 mM by diluting the cells with a non-selective growth medium (L-broth). The cells are held for approximately 1 hour in these conditions after which they are plated on selection media comprised of L-agar containing 100 ug per ml of ampicillin.

A number of ampicillin resistant colonies that grow up are replated on L-agar plates containing ampicillin and also on L-agar containing tetracycline. Colonies displaying ampicillin resistance and tetracycline senstitivity are retained, grown up and the plasmid is isolated using the Brinboim-cesium chloride method. The isolated plasmid is
again doubly-digested with Bam HI and Hind III using the method described
previously. Following concentration, the digests are resolved on a 6%
acrylamide gel to confirm fragment size. The fragments are run with known
size standards for markers. Two bands are obtained which are of the size
expected for the Mtx^rDHFR DNA sequence and the Bam HI/Hind III digested
pBR327. The plasmid thus obtained was desiganted pR11021 (ATCC 39398).


Preparation of Control Sequences - Promoter

Plasmid pTi A6 from A. tumefaciens was digested into various
fragments as described in Cell (March 1980), Vol. 19: 729, which is herein
incorporated by reference. Plasmid pNW31c-8-1 was obtained as described
in that article. pNW31c-8-1, which contains a 7.3 kb Bam HI restriction
endonuclease fragment, was further digested with XmaI, an isoschizomere of
SmaI, and BglII restriction endonucleases. The resulting mix of
XmaI/BglII fragments was cloned into plasmid pBen 15 which had been previously digested with XmaI and BglII restriction endonuclease. Plasmid
pBen15 is both chloramphenicol and kanamycin resistant, but the XmaI/BglII
digestion renders pBen15 kanamycin sensitive. The resulting plasmids were
transformed into LE392. The thus transformed LE392s were grown on selec-
tion medium containing chloramphenicol. Chloramphenicol resistant
colonies were counter-selected for kanamycin senstitivity. Those colonies
displaying chloramphenicol resistance and kanamycin sensitivity were grown
on selection media and the plasmids were obtained again by the Brinboim-
cesium chloride method. The plasmids thus obtained were digested with
XmaI and BglII and the fragments were resolved by agarose gel elecrophore-
sis as described above to confirm the size of the bands contained in the
plasmid. A plasmid thus obtained containing a 2.0 kb XmaI/BglII fragment
was designated pL5381 .


pL5381 was digested with Sau 3A 1 restriction endonuclease to
yield a 1.3 kb fragment having Sau 3A 1 ends. The 1.3 kb fragment is
derived from the XMAI/BglII fragment of pNW31C-8-1 that had been previous-
ly ligated into pL5381. This 1.3 kb fragment, having Sau 3A 1 ends, was
ligated into pBR327, which had been previously digested with Bam HI to
yield Bam HI "sticky" ends compatible with Sau 3A 1 ends. The ligation

0140504

was carried out with ligase salts (NEB), ATP (0.5 mM) and $T_4$Ligase to form plasmid pR100736 (ATCC 39400). LE392 was transformed with pR100736 under the transforming conditions described above. Transformants were selected for ampicillin resistance and tetracycline sensitivity. Plasmids from ampicillin resistant, tetracycline sensitive colonies were isolated by the Birnboim cesium chloride method, digested with Bam HI and run on a 0.7% agarose gel. A plasmid having a 1.3 kb fragment was isolated confirming the identify of pR100736.

The 1.3 kb Sau 3A1 fragment carried by pR100736 was used as the source of promoter sequences in the following manner. About 100 ug of pR100736 was digested with Sau 3A1 (NEB) in a buffer solution of 50 mM sodium chloride, 6 mM tris-HCl (pH 7.5), 5 mM magnesium chloride ($MgCl_2$), 100 ug per ml bovine serum albium (BSA). The digestion is carried out for about 3 hours at 30°C, followed by heat inactivation at 65°C for 3 minutes and quenching on ice. The digested fragments are purified by electrophoresis on a 1.5% agarose gel using a buffer of 40 mM Tris, 1 mM EDTA adjusted to pH 8 using glacial acetic acid. Size standards of TaqI digested pBR322 (NEB) are run on the same gel.

The purified 1.3 kb Sau 3A1 fragment is electroeluted, yielding about 30 ug of DNA. After two phenol extractions, this DNA is concentrated by ethanol precipitation to about 200 ug/ml. About 20 ug of the concentrated 1.3 kb fragment is resuspended in approximately 40 ul of a buffer containing 150 mM NaCl, 12 mM Tris-HCl, 12 mM mg/$Cl_2$, 6 mM $\beta$-mercaptoethanol, 100 ug/ml BSA and approximately 150 units of MnlI restriction endonuclease. The digestion was carried out for approximately 3 hours after which it was stopped by heat inactivation at 65°C for 3 minutes followed by quenching on ice. The resulting fragments were resolved by electrophoresis on a 2% agarose gel using a buffer of 40 mM Tris, 1 mM EDTA adjusted to pH 8.2 using glacial acetic. The electrophoresis was carried out at 100 to 150 volts for a period of time sufficient to resolve the fragments — between 2 and 5 hours. A 270 base pair fragment (0.27 kb) was obtained by electroelution. The electroeluted 270-base pair fragment was extracted by phenol twice and then concentrated by ethanol precipitation as described above.

The nucleotide sequence of a portion of the 270 base pair MNLI fragment has been determined. This portion includes 110 nucleotides comprising the 3' end of the 270 base pair sequence. The 110 nucleotides have the following sequence as determined by techniques well known to those skilled in the art (see Bethesda Research Laboratories (BRL), User Manual, M13 Cloning/Dideoxy Sequencing Manual© 1980, BRL, Inc., Gaithersburg, Maryland 20760 and Nu, N., and Messing, J., Gene (1982) 17:271-77).

```
        10        20        30        40        50        60        70
5'-AAGCTTGAAAATTAAGCCCCCCCCCGAAATCATCGCCACAGGTCGTCCCAGCCCGGCATCTATATATAGC
   HindIII                                                    ⌣⌣

        80        90        100       110       120       130       140
GCCAATATAGTTTGTCTTACACAAACACACCTCACATCAT-3'
                      ↑
```

The repeating nucleotide pair TA between the bracket is bleived to be the RNA polymerase recognition site and the region around the A indicated by the arrow is believed to be the "CAP" site. The nucleotide sequence of the region in the 5' direction from the HIND III site has not been determined.

Since the 270-base pair fragment obtained by MnlI digestion does not always yield blunt ends, blunt ends can be restored to the fragment by one of two methods. The fragment may be incubated with DNA polymerase I in 10 mM $\beta$-mercaptoethanol, 60 mM Tris chloride (pH 7.5), 8 mM magnesium chloride and 0.2 mM deoxyadenosine triphosphate (dATP), deoxythymidine triphosphate (dTTP), deoxyguanosine triphosphate (dGTP), and deoxycytidine triphosphate (dCTP). By using this method, missing deoxyribonucleotides are added to the 270-base pair fragment to yield blunt ends. Alternately, Mung bean nuclease (Biolabs) may be used to trim back unpaired 3' and 5' ends of the 270-base pair fragment to yield blunt ends.

### Preparation of Plasmid pR11021 for Ligation with the 270-base pair MnlI Fragment

Plasmid pR-11021 having therein the nucleotide sequence coding for Mtx$^r$DHFR is obtained by the Brinboim-cesium chloride method. About 50 ug of pR11021 is digested with about 150 units of Hind III restriction endonuclease in about 25 ul of 60 mM NaCl, 7 mM Tris, 7 mM $MgCl_2$, pH of

11    0140504

7.5 at 37°C. The DNA is concentrated by precipitation with ethanol. At this point, the plasmid pR11021 is a linear molecule with Hind III "sticky ends" having unpaired terminal bases at both ends. To complement the terminal bases of the linear pR11021 molecule, the molecule is incubated with about 30 units of Klenow fragment enzyme (Boerhinger Manheim), of E. coli DNA polymerase I in the presence of 0.02 mM each of dATP, dCTP, dTTP and dGTP, 20 mM $\beta$-mercaptoethanol, 60 mM Tris chloride at pH 7.5 and 8 mM magnesium chloride. After 3 hours at 16°C this mixture is heat inactivated at 65°C for 10-12 minutes and the DNA concentrated by ethanol precipitation. The DNA is resuspended in sterile water. The ligation of the linear pR11021 to the 270-base pair MnlI fragment is accomplished in a buffer containing 0.4 mM ATP, 30 mM Tris chloride at pH 7.5, 10 mM $MgCl_2$, 50 mM $Na_2Cl$, 10 mM $\beta$-mercaptoethanol, and 100 ug/ml BSA and approximately 200 units T4 DNA ligase is added. The ligation is conducted for approximately 24 hours at 20-22°C. The resulting ligation mixture contains a variety of free DNA species as well as partial and complete ligation products. At least one of the complete ligation products is comprised of the 270-base pair Mnl I fragment ligated into pR11021 to form a covalently closed ciruclar DNA plasmid vector desiqnated pR112048.

Transformation of E. coli with the pR112048 ligation products

100 ul of resuspended competent E. coli cells are added to all of the previously obtained pR112048 ligation mixture in a sterile glass tube. Transformation is conducted at 0°C for approximately 1 hour. The cells are then heat shocked at 37°C for 3 minutes, resuspended in 1.5 ml of L-broth and allowed to incubate at 37°C for approximately 1 hour after which the culture is plated out on L-agar medium plates containing 100 mg/ml ampicillin. Plates are incubated for a period of time sufficient to allow ampicillin resistant colonies to grow. Colonies containing plasmid pR112048 are determined by replicate plating and colony filter hybridization analysis using the 270-base pair MnlI fragment labeled with $^{32}$P by nick translation as a probe, essentially as described by Grunstein, M. and Hogness, D.S., (1979) P.N.A.S., USA, 72 3961 (1971); Rigby, P.W. et al., (1977), Mol. Biol. 13:237. Hybridization of the probe was detected by autoradiography on Kodak X-OMAT AR-5 film.

Determination of Orientation of the MnlI Fragment

Colonies hybridizing to the $^{32}$P labeled 270-base pair MnlI probe are cultured on L-agar with 100 ug/ml ampicillin and the plasmid is isolated by the Brinboim-cesium chloride method. To determine the orientation of the 270-base pair MnlI fragment in pR112048, the purified pR112048 is alternately digested with EcoRI and Hind III and SalI and Bam HI. The MnlI fragment is known to have a Hind III site approximately 160 base pairs from the 5' end thereof and therefore 110 bases from the 3' end thereof. An EcoRI site is known to be located approximately 30 base pairs in the 5' direction from the 5' end of the MnlI fragment. A Sal I site is known to exist approximately 665 base pairs in the 3' direction from the 3' terminus of the properly oriented MnlI fragment. Thus, the plasmid having the properly oriented MnlI fragment will yield an EcoRI/Bam HI fragment of approximately 490 base pairs and a Sal I one/Hind III fragment of approximately 766 base pairs. Improper orientation will be characterized by Sal I/Hind III fragment of approximately 816 base pairs. Other enzymes that can be used to test the construction are PstI/Hind III and Hind III/Bam HI. These double digests are run on a 5% acrylimide gel for between 3 to 5 hours in an appropriate buffer at approximately pH 8.0 and visualized by staining with ethidium bromide and exposure to ultraviolet light. Cells containing the plasmid that yield the predicted restriction endonuclease digestion fragments are grown up on L-broth.

Preparation of Nucleotide Sequence Containing Poly A Site for Ligation

Plasmid pL5351 is concentrated by ethanol precipitation to a working concentration of approximately 200 ug/ml and is suspended in Tris-EDTA buffer at pH 8.0. Approximately 40 ul of plasmid pL5351 are digested with SmaI and Hind III in a buffer of 20 mM KCl, 6 mM Tris-HCl (pH 8.0), 6 mM MgCl$_2$, 6 mM $\beta$-mercaptoethanol, 100 ug/ml BSA. After digestion for approximately 3 hours at 37°C, the digestion is terminated by heating to 65°C for 3 minutes followed by quenching on ice.

The SmaI-Hind III digest results in a 1.2 kb fragment that is resolved by electrophoresis in an agarose gel and removed by electroelution as described above. The 1.2 kb fragment is concentrated by ethanol precipitation and resuspended at a concentration of approximately 200 mg/ml in a Tris, EDTA buffer at approximately pH 8.0.

An aliquot of the 1.2 kb fragment is digested with Sau 3A1 in a buffer containing 50 mM NaCl, 6 mM Tris-HCl (pH 7.5), 5 mM $MgCl_2$ and 100 ug/ml BSA. The digestion is carried out at 30°C to completion. The digestion of the 1.2 kb fragment yields 4 fragments upon purification on a 2% agarose gel using a buffer of 40 mM Tris, 1 mM EDTA adjusted to pH 8 using glacial acetic acid. The 4 fragments are approximately 550-base pairs, approximately 480-base pairs, approximately 180-base pairs and approximately 8-base pairs in size. The 550-base pair fragment is electroeluted and concentrated by ethanol precipitation as described hereinabove to approximately 200 ug/ml and is used as a source of the Poly A signal sequence as described below.

The 480 base pair sequence is also known to possess promoterlike activity. This 480 base pair sequence which has one Hind III end and one Sau 3A1 end has the following nucleotide sequence:

```
        10        20        30        40        50        60        70
AAGCTTGAAAATTAAGCCCCCCCCCCGAAATCATCGCCACAGGTCGTCCCAGCCCGGCATCTATATATAGC
 HindIII

        80        90       100       110       120       130       140
GCCAATATAGTTTGTCTTACACAAACACACCTCACATCATGAATTTCGCAGATACTCCTTGGCCTCCCTC

       150       160       170       180       190       200       210
GACCTAGACTGGGCATGCGAAGAGTTTATCAAAACTTATGGTGCATCTCCACAATTGGAAACAGGAGAGG

       220       230       240       250       260       270       280
TAATCCAAACAAACAATGGGCTGCTGTATTTGTATGGCAAAGGTTCACTCTCACAGCGGATTCATGACAJ

       290       300       310       320       330       340       350
ACACCTCAAATTTAAGGAGAAGGAAGGATTATCCTTCACTACCATAAAGCCAGCTGAGATGAAGGCGCAA

       360       370       380       390       400       410       420
CAAAGTGATTTAACTTATTATGTCGCCATTTTTCAAAGCAACTATTTCCTGTGCGTTTCAAATCCAGAGA

       430       440       450       460       470       480
AAGGCTTTCTGAGA TGCCATAATCGCCCATTTCTGTACCCCATAGTAGCCCATGGATCC.
                                                       BamHI
```

It has been ligated into plasmid pBR327 previously digested with Bam HI and Hind III restriction endonucleases under conditions suggested by the supplier (NEB). The resultant plasmid designated pR4269 has been cloned into E. coli strain LE392 deposited under ATCC Accession Number 39397.

## Preparation of Plasmid Vector pR112048 for Ligation With DNA Sequence Coding Poly A Signal Site

The plasmid containing MnlI and MTX$^r$DHFR fragment is digested with Bam HI to yield a linear plasmid molecule having Bam HI "sticky" ends compatible with the Sau 3A1 "sticky" ends of the 550-base pair fragment. The 550-base pair fragment is heated to about 70-90°C and quenched on ice to remove secondary structures. The fragments are allowed to ligate overnight at about 15°C in a buffer containing 50 mM Tris-HCl (pH 7.8), 10 mM MgCl$_2$, 20 mM dithiothreitol, 0.5 uM ATP. 50 ug/ml BSA and about 150 units of T4DNA ligase (NEB). The entire ligation mixture is mixed with approximately 100 ul of E. coli strain LE392 containing about 10$^7$ competent cells. The transformation is carried out at 0°C for about an hour followed by heat shock at 37°C for about 3 minutes. The transformation mixture is diluted out with a non-selective growth medium until the calcium ion concentration is less than 20 mM. The cell colonies containing the 550-base pair fragment are identified by colony hybridization using $^{32}$P labeled 550-base pair fragment probes labeled by nick translation. Those colonies hybridizing to the $^{32}$P labeled 550-base pair fragments are plated out and the completed plasmid is isolated by the Brinboim-cesium chloride gradient method described above. The isolated plasmids are digested with Sau 3A1 and the digest fragments are resolved on a 5% acrylamide gel.

## Alternate Preparation of Poly A Containing DNA Sequence and Ligation With Plasmid Vector pR112048

The DNA containing the polyadenylation signals can also be obtained as follows. A 4.65 kb Bam HI fragment from pTiA6 previously cloned into pBr322 to form plasmid pBam 19 as described in Thomashaw et al., Cell, 19:729 (1980) is isolated by digestion with Bam HI and purified by electrophoresis. This 4.65 Bam HI fragment contains a PvuII fragment approximately 720 bp in length that has a polyadenylation site approximately 160 bp from one end. The plasmid containing this 4.65 kb Bam fragment is digested to completion with PvuII and the 720 bp PvuII fragment is purified by electrophoresis through an agarose gel as described previously. The fragment is concentrated after electroelution as described above.

The vector, pR112048, containing the promoter and DHFR gene is then digested to completion with Bam HI. The DNA is concentrated by ethanol precipitation and the unpaired bases at the Bam HI site are filled in by DNA Polymerase I Klenow fragment (NEB) as described hereinabove. The reaction is terminated by heating to 65°C and the blunt ended pR112048 plasmid is concentrated by ethanol precipitation. The ligation of linear pR112048 to the PvuII fragment is accomplished in ligation buffer as described above for 24 hours at 20-22°C, forming a ligation mixture.

This ligation mixture is used to transform competent LE392 as described above and the bacteria are plated on L-agar plates containing 100 ug/ml ampicillin. Ampicillin resistant colonies are selected for colony hybridization as before and those colonies having DNA homologous to probes comprised of $^{32}$P-labeled PvuII fragment are selected for further analysis.

Orientation of PvuIII Fragment

Those colonies with plasmid containing the PvuII fragment are grown in L-broth with ampicillin and the plasmid is isolated by the Brinboim-cesium chloride methods, then digested with RsaI. There is one RsaI site located asymmetrially within the PvuIII fragment. Digestion with this enzyme allows easy determination of orientation of the PvuII fragment plasmid. Plasmids containing the complete PvuII fragment in the proper 3'-5' orientation are designated pI11129. E. coli strain LE92 transformed with pI11129 are deposited under ATCC Accession Number 39396.

0140504

CLAIMS:

1.    A recombinant plasmid comprising a plasmid vector adapted for transformation of a host cell into which said plasmid vector a DNA sequence comprising a nucleotide sequence coding for a structural gene and control sequences for transcription and translation thereof have been inserted.

2.    The plasmid of Claim 1 wherein said plasmid vector is pBR327.

3.    The plasmid of Claim 1 wherein said structural gene is a marker.

4.    The plasmid of Claim 3 wherein said marker is a positive marker.

5.    The plasmid of Claim 4 wherein said marker is methotrexate resistant dihydrofolate reductase.

6.    The plasmid of Claim 1 wherein said control sequences include at least one promoter sequence and at least one terminator sequence.

7.    The plasmid of Claim 6 wherein said promoter functions in eukaryotic cells.

8.    The plasmid of Claim 7 wherein said cell is an eukaryotic cell.

9.    The plasmid of Claim 8 wherein said promoter is derived from a tumor inducing plasmid of Agrobacterium tumefaciens.

10.   The plasmid of Claim 9 wherein said tumor inducing plasmid is selected from the group consisting of pTiA6, pTiAch5 and pTiT37.

11. The plasmid of Claim 10 wherein said promoter is obtained from an MnlI fragment of plasmid pTiA6, the 3-terminal 110 bases of said MnlI fragment having the sequence:

```
        10        20        30        40        50        60        70
5'-AAGCTTGAAAATTAAGCCCCCCCCCGAAATCATCGCCACAGGTCGTCCCAGCCCGGCATCTATATATAGC
   HindIII

        80        90       100       110       120       130       140
GCCAATATAGTTTGTCTTACACAAACACACCTCACATCAT-3'
```

12. The plasmid of Claim 6 wherein said terminator sequence is a polyadenylation signal sequence.

13. The plasmid of Claim 17 wherein said polyadenylation signal sequence is derived from a tumor inducing plasmid of Agrobacterium tumefaciens.

14. The plasmid of Claim 13 wherein said tumor inducing plasmid is selected from the group consisting of pTiA6, pTiAch5 and pTiT37.

15. The plasmid of Claim 14 wherein said terminator sequence is obtained from a 720 bp Pvu II fragment, said Pvu II fragment being derived from a 4.65 bb Bam HI fragment of pTiA6.

16. The plasmid of Claim 1 wherein said host is prokaryotic.

17. The plasmid of Claim 1 wherein said host is eukaryotic.

18. The plasmid of Claim 17 wherein said host is at least one plant cell.

19. The plasmid of Claim 18 wherein said plant cell is a corn cell.

20. A recombinant plasmid comprising a plasmid vector adapted for transformation of a plant host, said plasmid vector comprising pBB327 having inserted therein a structural gene and control sequences for the transcription and translation thereof.

21. The plasmid of Claim 20 wherein said strucutral gene is a marker.

22. The plasmid of Claim 20 wherein said marker is methotrexate resistant dihydrofolate reductase.

23. The plasmid of Claim 20 wherein said control sequence includes at least one promoter sequence and at least one terminator sequence.

24. The plasmid of Claim 23 wherein said promotor functions in plant cells.

25. The plasmid of Claim 24 wherein said promoter is derived from a tumor inducing plasmid of Agerobacterium tumefaciens.

26. The plasmid of Claim 26 wherein said tumor inducing plasmid is selected from the group consisting of pTiA6, pTiAch5 and pTiT37.

27. The plasmid of Claim 26 wherein said promoter is an MnlI fragment of pTiA6, the 110 bases of said MnlI fragment having the sequence:

```
          10        20        30        40        50        60     p
                                                                     70
5'-AAGCTTGAAAATTAAGCCCCCCCCCGAAATCATCGCCACAGGTCGTCCCAGCCCGGCATCTATATATAGC
   HindIII

          80        90       100       110       120       130       140
GCCAATATAGTTTGTCTTACACAAACACACCTCACATCAT-3'
```

28. The plasmid of Claim 23 wherein said terminator sequence is a polyadenylation sequence.

29. The plasmid of Claim 28 wherein said polyadenylation sequence is derived from a tumor inducing plasmid of Agerobacterium tumefaciens.

30. The plasmid of Claim 29 wherein said tumor inducing plasmid is selected from the group consisting of pTiA6, pTiAch5 and pTiT37.

31. The plasmid of Claim 30 wherein said terminator sequence is obtained from a 720 pb Pvu II fragment, said Pvu II fragment being derived from a 4.65 kilobase Bam HI fragment of pTiA6.

32. The plasmid of Claim 27 wherein said terminator sequence is a polyadenylation sequence.

33. The plasmid of Claim 32 wherein said polyadeylation sequence is derived from a tumor-inducing plasmid of <u>Agrobacterium</u> <u>tumefaciens</u>.

34. The plasmid of Claim 33 wherein said terminator sequence is selected from the group consisting of pTiA6, pTiAch5 and pTiT37.

35. The plasmid of Claim 34 wherein said terminator sequence is obtained from a 720 bp Pvu II fragment, said Pvu II fragment being derived from a 4.65kilobase Bam HI fragment of pTiA6.

36. Plasmid pR112048.

37. The plasmid of Claim 36 as found in <u>E. coli</u> LE392 ATCC 39399.

38. Plasmid pR100736.

39. The plasmid of Claim 38 as found in <u>E. coli</u> LE392 ATCC 39400.

40. Plasmid pR11021.

41. The plasmid of Claim 40 as found in <u>E. coli</u> LE392 ATCC 39998.

42. Plasmid pI11129.

43. The plasmid of Claim 42 as found in <u>E. coli</u> LE392 ATCC 39396.

44. Plasmid pR4269.

0140504

45. The plasmid of Claim 44 as found in E. coli LE392 ATCC 39397.

46. The deoxoribonucleotide sequence:

```
       10        20        30        40        50        60        70
AAGCTTGAAAATTAAGCCCCCCCCCGAAATCATCGCCACAGGTCGTCCCAGCCCGGCATCTATATATAGC
 HindIII

       80        90       100       110       120       130       140
GCCAATATAGTTTGTCTTACACAAACACACCTCACATCATGAATTTCGCAGATACTCCTTGGCCTCCCTC

      150       160       170       180       190       200       210
GACCTAGACTGGGCATGCGAAGAGTTTATCAAAACTTATGGTGCATCTCCACAATTGGAAACAGGAGAGG

      220       230       240       250       260       270       280
TAATCCAAACAAACAATGGGCTGCTGTATTTGTATGGCAAAGGTTCACTCTCACAGCGGATTCATGACAJ

      290       300       310       320       330       340       350
ACACCTCAAATTTAAGGAGAAGGAAGGATTATCCTTCACTACCATAAAGCCAGCTGAGATGAAGGCGCAA

      360       370       380       390       400       410       420
CAAAGTGATTTAACTTATTATGTCGCCATTTTTCAAAGCAACTATTTCCTGTGCGTTTCAAATCCAGAGA

      430       440       450       460       470       480
AAGGCTTTCTGAGA TGCCATAATCGCCCATTTCTGTACCCCATAGTAGCCCATGGATCC.
                                                      BamHI
```

# Figure 1

## Figure 2

# Figure 2a

## Figure. 2b

Figure 3

**European Patent Office**

**EUROPEAN SEARCH REPORT**

| | DOCUMENTS CONSIDERED TO BE RELEVANT | | EP 84305535.1 |
|---|---|---|---|
| Category | Citation of document with indication, where appropriate, of relevant passages | Relevant to claim | CLASSIFICATION OF THE APPLICATION (Int. Cl.4) |
| X | SCIENCE, vol. 219, no. 4585, February 11, 1983, Washington (USA) R.A. HITZEMAN et al. "Secretion of Human Interferons by Yeast", pages 620-625 <br> * Pages 620,621 * <br> -- | 1,3,4, 6-8,12, 17 | C 12 N 15/00 <br> C 07 H 21/04 <br> //C 12 R 1/19 |
| X | EP - A2 - 0 068 740 (ELI LILLY) <br> * Columns 1,8,15,18 * <br> -- | 1,3,4, 6-8,18 | |
| D,A | JOURNAL OF MOLECULAR AND APPLIED GENETICS, vol. 1, no. 1, 1981, New York (USA) A. DEPICKER et al. "Nopaline Synthase: Transcript Mapping and DNA Sequence", pages 561-573 <br> * Pages 570-572 (Discussion) * <br> -- | 9,10, 12-14, 17,18 | |
| | | | TECHNICAL FIELDS SEARCHED (Int. Cl.4) |
| A,P | EP - A1 - 0 107 170 (YAMAMOTO) <br> * Column 5 * <br> -- | 2 | C 12 N <br> C 07 H |
| A | WO - A1 - 81/02 425 (AXEL) <br> * Columns 1,20,21 * <br> ---- | 1,3-8, 17 | |

The present search report has been drawn up for all claims

| Place of search | Date of completion of the search | Examiner |
|---|---|---|
| VIENNA | 23-11-1984 | FARNIOK |

EPO Form 1503 03.82